# EUROPÄISCHE PATENTSCHRIFT

(11) **EP 3 367 944 B1**
(45) Veröffentlichungstag und Bekanntmachung des Hinweises auf die Patenterteilung: **24.07.2019**
(21) Anmeldenummer: 16801389.4
(22) Anmeldetag: 31.10.2016
(51) Int. Cl.: A61B 18/14, A61B 17/00, A61B 17/3205

(54) **VORRICHTUNG ZUR AUFNAHME EINES PRÄPARATES IN EINEN BERGEBEUTEL**
DEVICE FOR RECEIVING SPECIMEN IN AN EXTRACTION BAG
DISPOSITIF POUR LA RÉCEPTION D'UNE PRÉPARATION DANS UN SAC D'EXTRACTION

(30) Priorität: 29.10.2015 DE 102015118545; 11.11.2015 DE 102015119427
(43) Veröffentlichungstag der Anmeldung: 05.09.2018
(73) Patentinhaber: BOWA-electronic GmbH & Co. KG, 72810 Gomaringen (DE)
(72) Erfinder: GRAF, Thomas, 72810 Gomaringen (DE); KIESSLING, Ingo, 72131 Ofterdingen (DE); NOÉ, Karl-Günter, 50769 Köln (DE); ANAPOLSKI, Michael, 40474 Düsseldorf (DE)
(74) Vertreter: Schneider, Peter Christian
(86) Internationale Anmeldenummer: PCT/EP2016/076233
(87) Internationale Veröffentlichungsnummer: WO 2017/072359

(56) Entgegenhaltungen:
- EP-A1- 0 739 604
- DE-A1- 10 328 329
- US-A- 5 190 542
- US-A- 6 004 330
- US-A1- 2007 016 225

## Beschreibung

### Gebiet der Erfindung

Die Erfindung betrifft eine Vorrichtung mit einem Bergebeutel zur Aufnahme eines Präparates und mit einer elektrochirurgischen Schneidschlinge, wobei das Präparat mit der elektrochirurgischen Schneidschlinge in einer Körperhöhle abgetrennt wurde, wobei der Bergebeutel an einem Ende eines in einem Einführungsrohr längsverschieblichen Aktuators angeordnet und aus einer distalen Öffnung des Einführungsrohres ausfahrbar ist, wobei im ausgefahrenem Zustand eine erste Beutelöffnung des Bergebeutels durch eine mit ihr verbundene elastisch federnde erste Schlinge aufgespannt und im eingefahrenem Zustand durch die erste Schlinge geschlossen und vom distalen Ende des Einführungsrohres abgedeckt ist, wobei die elektrochirurgische Schneidschlinge elastisch federnd ausgebildet und über den Aktuator mit dem Bergebeutel aus dem Einführungsrohr sich aufspannend ausfahrbar ist und wobei die Schneidschlinge der ersten Beutelöffnung benachbart angeordnet ist, und wobei die elektrochirurgische Schneidschlinge benachbart zu der ersten Schlinge angeordnet ist.

### Stand der Technik

Aus der US 2011/0184432 A1 ist eine Vorrichtung zur Aufnahme eines Präparates in einem Bergebeutel bekannt. Der Bergebeutel ist an einem Ende eines in einem Einführungsrohr längsverschieblich bewegbaren Aktuators angeordnet. Dabei ist der Bergebeutel aus einer zentralen Öffnung des Einführungsrohres ausfahrbar, wobei im ausgefahrenen Zustand eine erste Beutelöffnung des Bergebeutels durch eine mit ihr verbundene elastisch federnde erste Schlinge aufgespannt ist. Im eingefahrenen oder zurückgezogenen Zustand ist die Beutelöffnung durch die erste Schlinge geschlossen und vom distalen Ende des Einführungsrohres abgedeckt.

Nachteilig dabei ist, dass das zu bergende Präparat bzw. das abgetrennte Gewebe mit einer Zange aufgenommen und in den Bergebeutel eingebracht werden muss. Dies ist insbesondere auch dann nachteilig, wenn das abgetrennte Gewebe kanzerogen ist und dadurch in der Körperhöhle gestreut werden kann. Weiterhin kann beim Abtrennen des Präparats mit einer elektrochirurgischen Schneidschlinge in der Körperhöhle Rauchgas, das ggf. ebenfalls karzinogen ist, entstehen und sich ungehindert in der Körperhöhle ausbreiten.

Beispielsweise aus der DE 20 2007 006 619 U1 ist eine Vorrichtung mit einer elektrochirurgischen Schneidschlinge bekannt, die aus der Öffnung eines hohlen Schaftes in eine offene Stellung ausfahrbar und wieder in eine zusammengezogene Stellung einziehbar ist. Zum Schneiden ist die Schlinge als ein mit Hochfrequenzstrom beaufschlagbares monopolares Werkzeug ausgebildet.

Nachteilig bei der bekannten elektrochirurgischen Schneidschlinge ist, dass sie über das zu resezierende Präparat, beispielsweise Uterusgewebe positioniert und das Präparat durch Zuziehen der Schlinge unter Beaufschlagung mit Hochfrequenzstrom abgetrennt wird und in die Körperhöhle fällt, in der es mit einer Greifzange aufgenommen und in einen Bergebeutel durch eine Öffnung eingebracht wird. Bei karzinogenem Gewebe kann es dabei zu einer unerwünschten Streuung in der Körperhöhle kommen.

Weiterhin ist aus der US 5 618 296 A ein Vorrichtungssystem bekannt, bei dem in einer Körperhöhle reseziertes Gewebe in einen Bergebeutel eingebracht und mit einem sogenannten Morcellator innerhalb des Bergebeutels zerkleinert und abgesaugt wird. Ein ähnliches Vorrichtungssystem ist auch aus der US 6 162 235 A bekannt.

Auch bei diesen beiden Systemen besteht der Nachteil, dass das mit einer Schlinge zu resezierende Präparat in die Körperhöhle fällt und mithilfe einer Zange in den Bergebeutel eingebracht werden muss.

Weiterhin ist aus der US 5 190 542 A Vorrichtung zur Aufnahme eines Präparates in einen Bergebeutel, wobei das Präparat mit einer elektrochirurgischen Schneidschlinge in einer Körperhöhle abgetrennt wurde, bekannt. Dabei ist der Bergebeutel an einem Ende eines in einem Einführungsrohr längsverschieblichen Aktuators angeordnet und aus einer distalen Öffnung des Einführungsrohres ausfahrbar. Im ausgefahrenem Zustand ist eine erste Beutelöffnung des Bergebeutels durch eine mit ihr verbundene elastisch federnde erste Schlinge aufgespannt und im eingefahrenem Zustand ist die erste Schlinge geschlossen und vom distalen Ende des Einführungsrohres abgedeckt, wobei die elektrochirurgische Schneidschlinge elastisch federnd ausgebildet und über den Aktuator mit dem Bergebeutel aus dem Einführungsrohr sich aufspannend ausfahrbar ist. Dabei ist die Schneidschlinge der ersten Beutelöffnung benachbart angeordnet, wobei die elektrochirurgische Schneidschlinge auch benachbart zu der ersten Schlinge angeordnet ist.

### Aufgabenstellung

Es ist die Aufgabe der vorliegenden Erfindung, die bekannten Vorrichtungen so zu verbessern, dass ein direkter Kontakt des resezierten Präparats mit der Umgebung der Körperhöhle vermieden wird. Gleichzeitig soll das sichere Einbringen des Präparats in den Bergebeutel und zugleich ein sicheres Entfernen des Präparats und des Bergebeutels erleichtert werden.

### Darlegung der Erfindung

Die Aufgabe wird in Verbindung mit den Merkmalen des Oberbegriffs von Anspruch 1 dadurch gelöst, dass die Schneidschlinge an ihrem distalen Ende einen freiliegenden, unisolierten, elektrisch leitenden Schneidbereich aufweist und in einem Isolierbereich außerhalb des Schneidbereiches isoliert ausgebildet ist, dass der Bergebeutel mindestens eine zweite verschließbare Beutelöffnung aufweist, und dass die zweite Beutelöffnung an dem der ersten Beutelöffnung abgewandten Ende des Bergebeutels angeordnet ist.

Dadurch, dass die elektrochirurgische Schneidschlinge über den Aktuator zusammen mit dem Bergebeutel aus dem Einführungsrohr sich aufspannend ausfahrbar ist und die Schneidschlinge zudem der ersten Beutelöffnung benachbart angeordnet ist, lässt sich die Schneidschlinge zusammen mit der Beutelöffnung über das zu resezierende Präparat stülpen, so dass beim Zuziehen der Schneidschlinge und durch Trennen des Präparats das Präparat direkt in den Bergebeutel fällt, dessen Beutelöffnung durch die erste Schlinge verschlossen wird, so dass sich auch evtl. Rauchgas im Beutel befindet. Eine Ausbreitung von kanzerogen reseziertem Gewebe und auch von Rauchgas in der Körperhöhle wird dadurch zuverlässig vermieden.

Unter benachbart soll dabei direkt oder nur mit geringem Abstand benachbart verstanden werden. Durch den geringen Abstand zwischen der elektrochirurgische Schneidschlinge und der ersten Schlinge kann die im Einführungsrohr benötigte lichte Weite für die beiden Schlingen und den Bergebeutel relativ klein gehalten werden. Die Schneidschlinge kann zudem unabhängig von der ersten Schlinge der Beutelöffnung zugezogen werden, wobei das Gewebe durchtrennt wird und das Präparat in den Bergebeutel fällt. Anschließend kann durch die erste Schlinge die Beutelöffnung zugezogen werden.
Durch die Isolierung außerhalb des Schneidbereiches wird sichergestellt, dass im restlichen Isolationsbereich - also außerhalb des Schneidbereiches - kein unbeabsichtigtes Schneiden möglich ist.

Die zweite Beutelöffnung ist an dem der ersten Öffnung abgewandten Ende des Bergebeutels angeordnet. Durch zweite Beutelöffnung kann beispielsweise eine Kamera zur Kontrolle der Morcellation (Zerkleinerung des resezierten Präparats) in den Beutel eingebracht werden. Die zweite Beutelöffnung kann dabei als ein verschließbarer Port ausgebildet sein. Es ist aber auch möglich, dass die zweite Beutelöffnung als ein verschließbarer und /oder ein öffnungsfähiger Port ausgebildet ist.

Gemäß einer bevorzugten Ausführungsform der Erfindung ist die elektrochirurgische Schneidschlinge als unipolare oder bipolare Schneidelektrode ausgebildet, die mit einem Hochfrequenzgenerator verbindbar ist. Durch die Schneidelektrode wird das Gewebe durchtrennt und gleichzeitig koaguliert, so dass Blutungen vermieden werden. Grundsätzlich kann die elektrochirurgische Schneidschlinge auch als eine bipolare Schneidelektrode ausgebildet sein.

Gemäß einer weiteren bevorzugten Ausführungsform der Erfindung wird die elektrochirurgische Schneidschlinge von der ersten Schlinge gebildet. Durch die Verwendung nur einer Schlinge sowohl zum Öffnen und Verschließen der Beutelöffnung als auch zum Resezieren bzw. Abtrennen von Gewebe kann der Querschnitt des Einführungsrohres relativ gering gehalten werden. Zudem ist der Aufbau der Vorrichtung relativ einfach und kostengünstig. Die elektrochirurgische Schneidschlinge wird gleichzeitig mit dem daran befestigten Bergebeutel zugezogen und das Gewebe durchtrennt, wobei das Präparat im Bergebeutel verbleibt.

Nach einer weiteren bevorzugten Ausführungsform der Erfindung ist die elektrochirurgische Schneidschlinge über Abstandshalter mit der ersten Schlinge verbunden. Durch die Abstandshalter wird sichergestellt, dass der Abstand zwischen erster Schlinge und Schneidschlinge einen Maximalabstand nicht übersteigt und kein Gewebe zwischen den Schlingen austreten kann. Zudem wird sichergestellt, dass die Beutelöffnung nicht vor dem Abtrennen des Gewebes verschlossen wird. Durch die Abstandshalter wird somit auch eine koordinierte Bewegung zwischen der ersten Schlinge und der elektrochirurgischen Schneidschlinge sichergestellt.

Nach einer weiteren bevorzugten Ausführungsform der Erfindung ist die erste Schlinge lösbar mit dem Bergebeutel verbunden. Dies kann insbesondere außerhalb der Körperhöhle geschehen, um beispielsweise einen Morcellator (Zerkleinerungsvorrichtung) durch die Beutelöffnung in den noch in der Körperhöhle verbliebenen Beutelbereich zur Zerkleinerung des Gewebes einzubringen.

Die zweite Beutelöffnung ist nach einer weiteren bevorzugten Ausführungsform der Erfindung durch Zuziehen eines im Öffnungsbereich angeordneten Verschlussbandes verschließbar. Die doppelte Öffnung des Beutels ermöglicht ein perforationsfreies Einführen sowohl des Morcellators als auch einer Kamera. Die doppelte Öffnung des Beutels ermöglicht auch eine Abdichtung des Beutels zur Körperhöhe bzw. zum Bauchraum hin. Zur besseren Sicht ist dabei ein Aufblasen des Beutels möglich. Ein unerwünschter Druckverlust im Bergebeutel kann dabei gering gehalten werden, was wiederum mit einer CO₂-Einsparung verbunden sein kann.

Gemäß einer weiteren bevorzugten Ausführungsform der Erfindung ist die Schneidschlinge mit dem Bergebeutel lösbar verbunden. Dies kann insbesondere außerhalb der Körperhöhle geschehen, um beispielsweise einen Morcellator (Zerkleinerungsvorrichtung) durch die Beutelöffnung in den noch in der Körperhöhle verbliebenen Beutelbereich zur Zerkleinerung des Gewebes einzubringen.

Nach einer weiteren bevorzugten Ausführungsform der Erfindung ist der Bergebeutel mindestens im Bereich der Schneidschlinge aus thermisch beständigen Material ausgebildet.
Auch die Isolationsschicht der Schneidschlinge ist bevorzugt aus einem thermisch beständigen Material ausgebildet.
Als thermisch beständiges Material eignet sich beispielsweise ein Polytetrafluoräthylen (PTFE), das auch modifiziert sein kann, oder ein Fluoräthylen-Propylen (FEP).

### Kurzbeschreibung der Zeichnungen

Es zeigen:
- Figur 1:: eine Draufsicht im Ausriss und vergrößerter Darstellung auf eine Vorrichtung zur Aufnahme eines Präparates in einem Bergebeutel mit ausgefahrener elektrochirurgischer Schneidschlinge als erster mit der ersten Beutelöffnung verbundenen Schlinge;
- Figur 2:: eine Draufsicht im Ausriss und vergrößerter Darstellung auf eine Vorrichtung zur Aufnahme eines Präparates in einem Bergebeutel mit ausgefahrener elektrochirurgischer Schneidschlinge, die benachbart zu der ersten mit der ersten Beutelöffnung verbundenen Schlinge angeordnet ist
- Figur 3:: eine Seitenansicht der Vorrichtung von Figur 1 in nicht ausgefahrenem Zustand eingeführt über eine Trokarhülse in einer Körperhöhle (im Ausriss);
- Figur 4:: eine Seitenansicht der Vorrichtung von Figur 3 aus Richtung IV im ausgefahrenen Zustand von Schneidschlinge und Bergebeutel mit geöffneter erster über das zu resezierende Präparat gestülpter Beutelöffnung;
- Figur 5:: eine Seitenansicht der Vorrichtung von Figur 4 im eingefahrenen Zustand der Schneidschlinge und mit geschlossener erster Beutelöffnung und in dem Bergebeutel angeordneten resezierten Präparat und
- Figur 6:: eine Seitenansicht des Bergebeutels von Figur 5, dessen Enden aus den Körperzugangsöffnungen für die Trokarhülsen zur weiteren Bearbeitung herausgezogen wurden.

### Beschreibung bevorzugter Ausführungsformen

Eine Vorrichtung 1 zur Aufnahme eines Präparates 2 besteht im Wesentlichen aus einem Einführungsrohr 3, einem Aktuator 4, einem Bergebeutel 5 und einer elektrochirurgischen Schneidschlinge 6.

Das Einführungsrohr 3 weist in seinem zentralen Führungskanal den längsverschieblichen Aktuator 4 auf. Am distalen Ende 7 des Aktuators 4 ist eine elastisch federnde erste Schlinge 8 angeordnet, die mit einer ersten Beutelöffnung 9 des Bergebeutels 5 verbunden ist und im aus der distalen Öffnung 10 des Einführungsrohres 3 ausgefahrenem Zustand des distalen Endes 7 die erste Beutelöffnung 9 aufspannt. Im noch nicht ausgefahrenen zurückgezogenen Zustand des Aktuators 4 sind die Schlingen 6, 8 mit dem Bergebeutel 5 komplett von dem distalen Ende 11 des Einführungsrohres 3 abgedeckt. Die erste Beutelöffnung 9 ist an einem der ersten Schlinge 8, 8' zugewandten ersten Ende 18 des Bergebeutels 5 angeordnet.

Die elektrochirurgische Schneidschlinge 6 ist elastisch federnd ausgebildet, so dass sie sich in den ausgefahrenen Zustand aufspannt, also durch ihre Federkraft sich öffnet. Gleiches gilt, wie oben beschrieben, auch für die erste Schlinge 8.

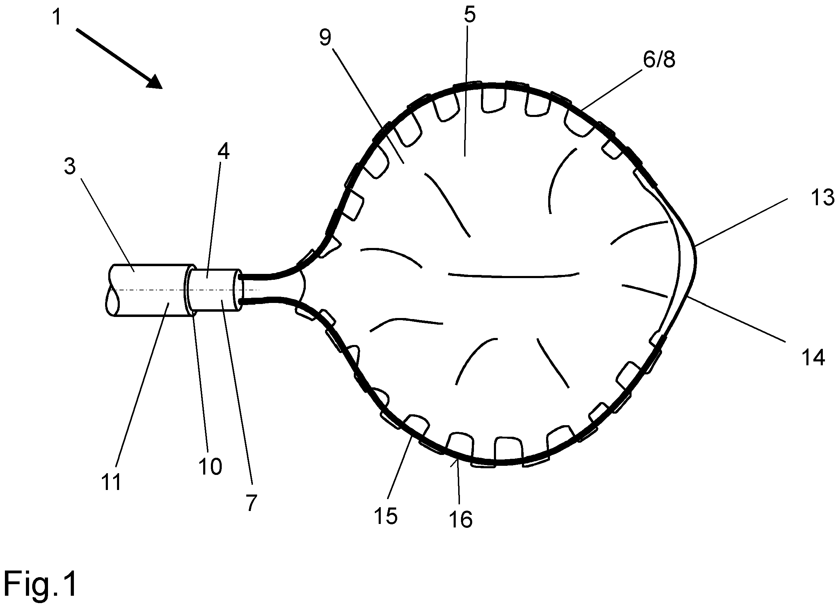

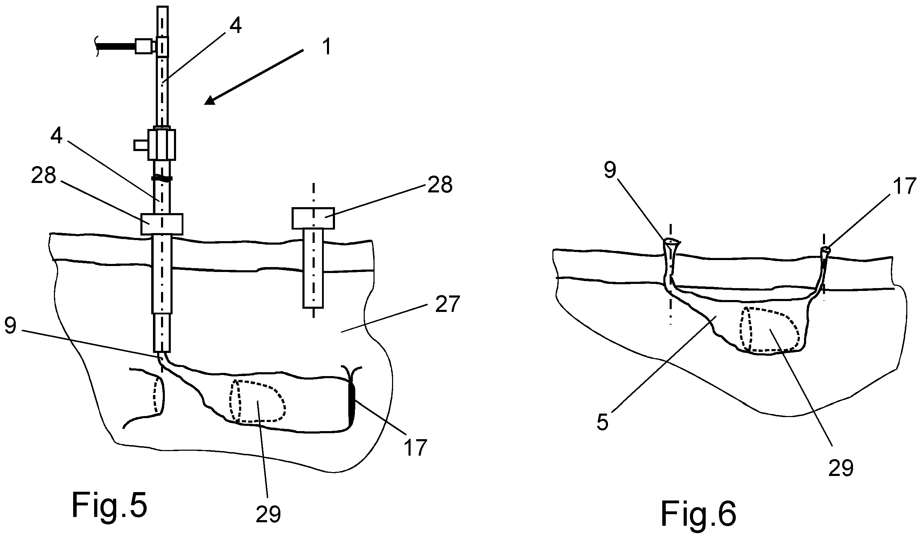

Entsprechend den Figuren 1 und 3 bis 6 wird die elektrochirurgische Schneidschlinge 6 von der ersten Schlinge 8 gebildet.

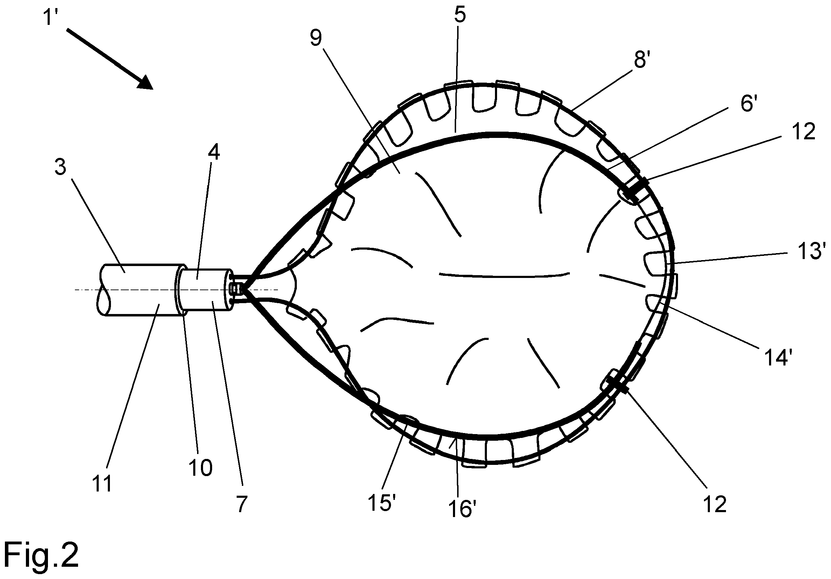

Entsprechend Figur 2 ist die elektrochirurgische Schneidschlinge 6' benachbart zu der ersten Schlinge 8' angeordnet. Die elektrochirurgische Schneidschlinge 6' ist dabei über Abstandshalter 12 mit der ersten Schlinge 8' verbunden. Grundsätzlich kann aber auch auf die Abstandshalter 12 verzichtet werden.

Die elektrochirurgische Schneidschlinge 6, 6' weist an ihrem distalen Ende 13, 13' einen freiliegenden, unisolierten, elektrisch leitenden Schneidbereich 14, 14' auf und ist außerhalb des Schneidbereiches 14, 14' in einem Isolationsbereich 15, 15' mit einer Isolationsschicht 16, 16', beispielsweise einem Kunststoffschlauch, versehen. Im Schneidbereich 14, 14' kann die Schneidschlinge stärker gekrümmt sein als außerhalb des Schneidbereiches.

Die erste Schlinge 8, 8' ist lösbar mit dem Bergebeutel 5 verbunden.
Entsprechend den Ausführungsbeispielen ist die elektrochirurgische Schneidschlinge 6, 6' lösbar mit dem Bergebeutel 5 verbunden.

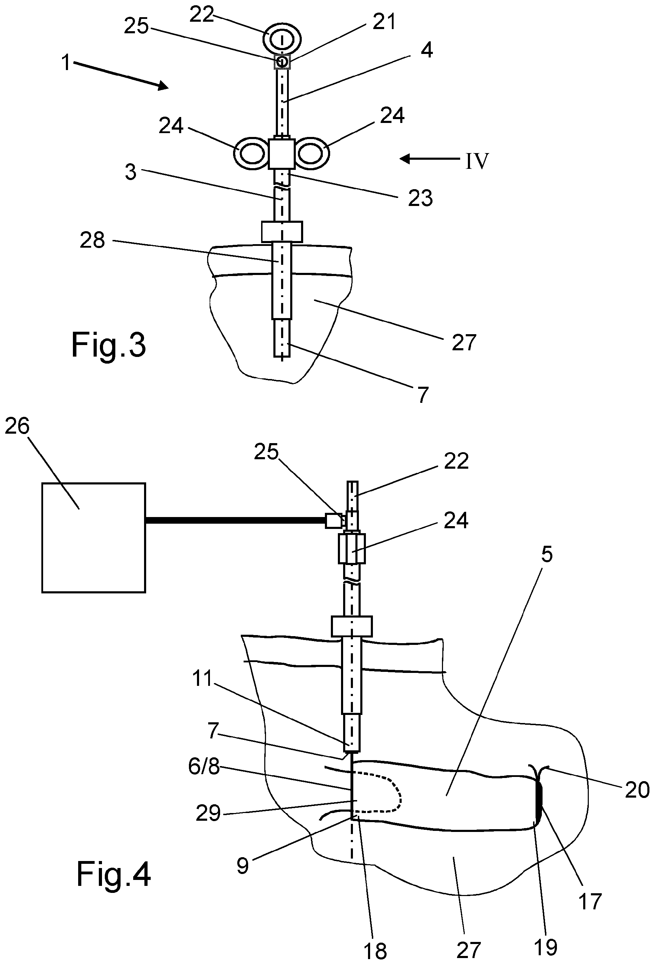

Der Bergebeutel 5 weist eine verschließbare zweite Beutelöffnung 17 auf. Die zweite Beutelöffnung 17 ist entsprechend den Figuren 4 bis 6 an dem den ersten Ende 18 mit der ersten Beutelöffnung 9 abgewandten zweiten Ende 19 des Bergebeutels5 angeordnet. Die zweite Beutelöffnung 17 ist durch Zuziehen eines im Öffnungsbereich der zweiten Beutelöffnung 17 angeordneten Verschlussbandes 20 verschließbar. Die zweite Beutelöffnung 17 kann auch als ein verschließbarer und/oder ein öffnungsfähiger Port ausgebildet sein.

Entsprechend den Figuren 3 bis 5 weist der Aktuator 4 an seinem proximalen Ende 21 einen Daumenring 22 zur Längsbewegung des Aktuators 4 gegenüber dem Einführungsrohr 3 auf. Das Einführungsrohr 3 weist an seinem proximalen Ende 23 zwei Halteringe 24 zur Aufnahme beispielsweise eines Zeige- und eines Mittelfingers auf.

An seinem proximalen Ende 21 weist der Aktuator 4 einen Hochfrequenzanschluss 25 zur Verbindung mit einem elektrochirurgischen Hochfrequenzgenerator 26 auf.

Zum Einführen des Einführungsrohres 3 in eine Körperhöhle 27 wird das distale Ende 11 über einen dem Fachmann bekannten (Laparoskop-) Trokar 28, der in die Körperhöhle 27 eingebracht wurde, eingeführt. Der Aktuator 4 wird in einem Folgeschritt betätigt und mit seinem distalen Ende 7 ausgefahren, so dass sich die die Schneidschlinge 6 und die erste Schlinge 8 öffnet und die Schlingen 6, 8 zusammen mit der ersten Beutelöffnung 9 über das zu resezierende Präparat 29 gestülpt werden. In einem weiteren Folgeschritt wird die Schneidschlinge 6 zugezogen, d. h. über den Aktuator 4 in das Einführungsrohr 3 zurückgezogen, und dabei das Präparat durchtrennt, so dass es durch die erste Beutelöffnung 9 in den Bergebeutel fällt. Dabei wird gleichzeitig oder in einem zweiten Arbeitsgang die erste Beutelöffnung durch die erste Schlinge 8 verschlossen. In einem weiteren Folgeschritt kann das erste Ende 18 des Bergebeutels 5 außerhalb der Körperhöhle 27 gebracht werden. Das zweite Ende 19 des Bergebeutels 5 kann über eine durch einen zweiten Trokar 28 eingeführte nicht dargestellte Fasszange erfasst und ebenfalls außerhalb der Körperhöhle 27 gebracht werden.

In weiteren Folgeschritten kann durch die erste Beutelöffnung 9 ein nicht dargestellter Morcellator und durch die zweite Beutelöffnung 17 beispielsweise eine nicht dargestellte Kamera in den Bergebeutel 5 eingebracht werden. Das Präparat 29 kann im Bergebeutel 5 morcelliert (zerkleinert) und aus der Körperhöhle 27 (innerhalb des Beutels) entfernt werden.

Natürlich stellen die in der speziellen Beschreibung diskutierten und in den Figuren gezeigten Ausführungsformen nur illustrative Ausführungsbeispiele der vorliegenden Erfindung dar. Dem Fachmann ist im Lichte der hiesigen Offenbarung ein breites Spektrum von Variationsmöglichkeiten an die Hand gegeben. Insbesondere kann die Relativbewegung zwischen dem Einführungsrohr 3 und dem Aktuator 4 am proximalen Ende 21 des Aktuators 4 und an dem proximalen Ende 23 des Einführungsrohres 3 über nicht dargestellte gegeneinander verschiebbare Griffstücke erfolgen.

### Bezugszeichenliste

- 1: Vorrichtung
- 2: Präparat
- 3: Einführungsrohr
- 4: Aktuator
- 5: Bergebeutel
- 6, 6': elektrochirurgische Schneidklinge
- 7: distales Ende von 4
- 8, 8': erste Schlinge
- 9: erste Beutelöffnung von 5
- 10: distale Öffnung von 3
- 11: distales Ende von 3
- 12: Abstandshalter
- 13, 13': distales Ende von 6, 6'
- 14, 14': unisolierter Schneidbereich von 13, 13'
- 15, 15': Isolierbereich von 6, 6'
- 16, 16': Isolationsschicht von 15, 15'
- 17: zweite Beutelöffnung von 5
- 18: erstes Ende von 5
- 19: zweites Ende von 5
- 20: Verschlussband von 17
- 21: proximales Ende von 4
- 22: Daumenring von 4
- 23: proximales Ende von 3
- 24: Haltering von 3
- 25: Hochfrequenzanschluss von 4
- 26: Hochfrequenzgenerator
- 27: Körperhöhle
- 28: Trokar
- 29: Präparat

## Patentansprüche

1. Vorrichtung (1) mit einem Bergebeutel (5) zur Aufnahme eines Präparates (29) und mit einer elektrochirurgischen Schneidschlinge (6, 6'), wobei das Präparat (29) mit der elektrochirurgischen Schneidschlinge (6, 6') in einer Körperhöhle (27) abgetrennt wurde, wobei der Bergebeutel (5) an einem Ende (7) eines in einem Einführungsrohr (3) längsverschieblichen Aktuators (4) angeordnet und aus einer distalen Öffnung (10) des Einführungsrohres (3) ausfahrbar ist, wobei im ausgefahrenem Zustand eine erste Beutelöffnung (9) des Bergebeutels (5) durch eine mit ihr verbundene elastisch federnde erste Schlinge (8, 8') aufgespannt und im eingefahrenem Zustand durch die erste Schlinge (8, 8') geschlossen und vom distalen Ende (11) des Einführungsrohres (3) abgedeckt ist,
wobei die elektrochirurgische Schneidschlinge (6, 6') elastisch federnd ausgebildet und über den Aktuator (4) mit dem Bergebeutel (5) aus dem Einführungsrohr (3) sich aufspannend ausfahrbar ist und
wobei die Schneidschlinge (6, 6') der ersten Beutelöffnung (9) benachbart angeordnet ist, und wobei die elektrochirurgische Schneidschlinge (6') benachbart zu der ersten Schlinge (8') angeordnet ist,
**dadurch gekennzeichnet,**
**dass** die Schneidschlinge (6, 6') an ihrem distalen Ende (13, 13') einen freiliegenden, unisolierten, elektrisch leitenden Schneidbereich (14, 14') aufweist und in einem Isolierbereich (15, 15') außerhalb des Schneidbereiches (14, 14') isoliert ausgebildet ist,
**dass** der Bergebeutel (5) mindestens eine zweite verschließbare Beutelöffnung (17) aufweist, und
**dass** die zweite Beutelöffnung (17) an dem der ersten Beutelöffnung (9) abgewandten Ende des Bergebeutels (5) angeordnet ist.

2. Vorrichtung nach Anspruch 1,
**dadurch gekennzeichnet,**
**dass** die elektrochirurgische Schneidschlinge (6, 6') als unipolare oder bipolare Schneidelektrode ausgebildet und mit einem Hochfrequenzgenerator (26) verbindbar ist.

3. Vorrichtung nach Anspruch 1 oder 2,
**dadurch gekennzeichnet,**
**dass** die elektrochirurgische Schneidschlinge (6') über Abstandshalter (12) mit der ersten Schlinge (8') verbunden ist.

4. Vorrichtung nach einem der Ansprüche 1 bis 3,
**dadurch gekennzeichnet,**
**dass** die erste Schlinge (8, 8') lösbar mit dem Bergebeutel (5) verbunden ist.

5. Vorrichtung nach einem der Ansprüche 1 bis 4,
**dadurch gekennzeichnet,**
**dass** die zweite Beutelöffnung (17) als ein verschließbarer Port ausgebildet ist.

6. Vorrichtung nach einem der Ansprüche 1 bis 4,
**dadurch gekennzeichnet,**
**dass** die zweite Beutelöffnung (17) als ein öffnungsfähiger Port ausgebildet ist.

7. Vorrichtung nach einem der Ansprüche 1 bis 4,
**dadurch gekennzeichnet,**
**dass** die zweite Beutelöffnung (17) durch Zuziehen eines im Öffnungsbereich angeordneten Verschlussbandes (20) verschließbar ist.

8. Vorrichtung nach einem der Ansprüche 1 bis 7,
**dadurch gekennzeichnet,**
**dass** die Schneidschlinge (6, 6') mit dem Bergebeutel (5) lösbar verbunden ist.

9. Vorrichtung nach einem der Ansprüche 1 bis 8,
**dadurch gekennzeichnet,**
**dass** der Bergebeutel (5) mindestens im Bereich der Schneidschlinge (6, 6') aus einem thermisch beständigen Material ausgebildet ist.

10. Vorrichtung nach einem der Ansprüche 1 bis 9,
**dadurch gekennzeichnet,**
**dass** die Isolationsschicht (16, 16') der Schneidschlinge (6, 6') aus einem thermisch beständigen Material ausgebildet ist.

11. Vorrichtung nach Anspruch 9 oder 10,
**dadurch gekennzeichnet,**
**dass** das thermisch beständige Material ein Polytetrafluoräthylen ist.

12. Vorrichtung nach Anspruch 9 oder 10,
**dadurch gekennzeichnet,**
**dass** das thermisch beständige Material ein Fluoräthylen-Propylen ist.

## Claims

1. A device (1) having an extraction bag (5) for receiving a preparation (29) and having an electrosurgical cutting loop (6, 6'), the preparation (29) having been detached using the electrosurgical cutting loop (6, 6') in a body cavity (27), wherein the extraction bag (5) is arranged at one end (7) of an actuator (4) which can be moved longitudinally in an insertion tube (3), and the extraction bag (5) can be extended out of a distal opening (10) of the insertion tube (3), wherein, in the extended state, a first bag opening (9) of the extraction bag (5) is held open by an elastically flexible first loop (8, 8') which is connected to the extraction bag, and, in the retracted state, the extraction bag is closed by the first loop (8, 8') and covered by the distal end (11) of the insertion tube (3),
wherein the electrosurgical cutting loop (6, 6') is designed to be elastically flexible and can be extended out of the insertion tube (3) together with the extraction bag (5) via the actuator (4) in an expanding manner, and
wherein the cutting loop (6, 6') is arranged adjacent to the first bag opening (9), and the electrosurgical cutting loop (6') is arranged adjacent to the first loop (8'),
**characterized in that**
the cutting loop (6, 6') has an exposed non-insulated electrically conductive cutting region (14, 14') at the distal end (13, 13') of the loop, and the cutting loop is insulated in an insulated region (15, 15') outside of the cutting region (14, 14'),
the extraction bag (5) has at least one second closable bag opening (17), and the second bag opening (17) is arranged at the extraction bag (5) end facing away from the first bag opening (9).

2. The device according to claim 1,
**characterized in that**
the electrosurgical cutting loop (6, 6') is a unipolar or bipolar cutting electrode and is connectable to a high-frequency generator (26).

3. The device according to claim 1 or 2,
**characterized in that**
the electrosurgical cutting loop (6') is connected to the first loop (8') via spacers (12).

4. The device according to any of claims 1 to 3,
**characterized in that**
the first loop (8, 8') is detachably connected to the extraction bag (5).

5. The device according to any of claims 1 to 4,
**characterized in that**
the second bag opening (17) is a closable port.

6. The device according to any of claims 1 to 4,
**characterized in that**
the second bag opening (17) is an openable port.

7. The device according to any of claims 1 to 4,
**characterized in that**
the second bag opening (17) is closable by pulling closed a closure band (20) arranged in the opening region.

8. The device according to any of claims 1 to 7,
**characterized in that**
the cutting loop (6, 6') is detachably connected to the extraction bag (5).

9. The device according to any of claims 1 to 8,
**characterized in that**
the extraction bag (5) is made of a thermally resistant material, at least in the region of the cutting loop (6, 6').

10. The device according to any of claims 1 to 9,
**characterized in that**
the insulation layer (16, 16') of the cutting loop (6, 6') is made of a thermally resistant material.

11. The device according to claim 9 or 10,
**characterized in that**
the thermally resistant material is a polytetrafluoroethylene.

12. The device according to claim 9 or 10,
**characterized in that**
the thermally resistant material is a fluorinated ethylene propylene.

## Revendications

1. Dispositif (1) avec un sac d'extraction (5) pour la réception d'une préparation (29) et avec une boucle de coupe (6, 6') électro-chirurgicale, la préparation (29) ayant été séparée par la boucle de coupe (6, 6') électro-chirurgicale dans une cavité corporelle (27), le sac d'extraction (5) étant disposé à une extrémité (7) d'un actionneur (4) coulissant dans le sens longitudinal dans un tube d'introduction (3) et étant extractible par une ouverture distale (10) du tube d'introduction (3), une première ouverture de sac (9) du sac d'extraction (5) étant tendue par une première boucle (8, 8') élastique à laquelle elle est reliée quand le sac est extrait et étant fermée par la première boucle (8, 8') et recouverte par l'extrémité distale (11) du tube d'introduction (3) quand le sac est rentré,
la boucle de coupe (6, 6') électro-chirurgicale ayant une configuration élastique et pouvant être extraite hors du tube d'introduction (3) avec le sac d'extraction (5) par l'intermédiaire de l'actionneur (4) en se tendant et
la boucle de coupe (6, 6') étant en position adjacente à la première ouverture de sac (9), et la boucle de coupe (6') électro-chirurgicale étant en position adjacente à la première boucle (8'),
**caractérisé**
**en ce que** la boucle de coupe (6, 6') présente à son extrémité distale (13, 13') une zone de coupe (14, 14') électriquement conductrice, non isolée et saillante, et est isolée dans une zone d'isolation (15, 15') en dehors de la zone de coupe (14, 14'), en ce que le sac d'extraction (5) présente au moins une seconde ouverture de sac (17) pouvant se fermer, et
**en ce que** la seconde ouverture de sac (17) est disposée à l'extrémité du sac d'extraction (5) opposée à la première ouverture de sac (9).

2. Dispositif selon la revendication 1,
**caractérisé**
**en ce que** la boucle de coupe (6, 6') électro-chirurgicale est réalisée sous forme d'électrode de coupe unipolaire ou bipolaire et peut être reliée à un générateur haute fréquence (26).

3. Dispositif selon la revendication 1 ou 2,
**caractérisé**
**en ce que** la boucle de coupe (6') électro-chirurgicale est reliée à la première boucle (8') par l'intermédiaire d'entretoises (12).

4. Dispositif selon l'une quelconque des revendications 1 à 3,
**caractérisé**
**en ce que** la première boucle (8, 8') est reliée de manière détachable au sac d'extraction (5).

5. Dispositif selon l'une quelconque des revendications 1 à 4,
**caractérisé**
**en ce que** la seconde ouverture de sac (17) est réalisée sous la forme d'un port pouvant être fermé.

6. Dispositif selon l'une quelconque des revendications 1 à 4,
**caractérisé**
**en ce que** la seconde ouverture de sac (17) est réalisée sous la forme d'un port pouvant être ouvert.

7. Dispositif selon l'une quelconque des revendications 1 à 4,
**caractérisé**
**en ce que** la seconde ouverture de sac (17) peut être fermée en tirant pour la fermer une bande de fermeture (20) disposée dans la zone d'ouverture.

8. Dispositif selon l'une quelconque des revendications 1 à 7,
**caractérisé**
**en ce que** la boucle de coupe (6, 6') est reliée de manière détachable au sac d'extraction (5).

9. Dispositif selon l'une quelconque des revendications 1 à 8,
**caractérisé**
**en ce que** le sac d'extraction (5) est réalisé au moins dans la zone de la boucle de coupe (6, 6') en un matériau thermiquement résistant.

10. Dispositif selon l'une quelconque des revendications 1 à 9,
**caractérisé**
**en ce que** la couche d'isolation (16, 16') de la boucle de coupe (6, 6') est réalisée en un matériau thermiquement résistant.

11. Dispositif selon la revendication 9 ou 10,
**caractérisé**
**en ce que** le matériau thermiquement résistant est un polytétrafluoréthylène.

12. Dispositif selon la revendication 9 ou 10,
**caractérisé**
**en ce que** le matériau thermiquement résistant est un fluoroéthylène-propylène.
